# FASCICULE DE BREVET EUROPEEN

(11) **EP 0 721 947 B1**
(45) Date de publication et mention de la délivrance du brevet: **14.03.2001**
(21) Numéro de dépôt: 96400052.5
(22) Date de dépôt: 10.01.1996
(51) Int. Cl.: C07D 307/81, A61K 31/343

(54) **Nouveaux composés (hétéro)cycliques alkylés, leur procédé de préparation et les compositions pharmaceutiques qui les contiennent**
Alkyl(hetero)cyclische Derivate, Verfahren zu deren Herstellung und sie enthaltende pharmazeutische Zusammensetzungen
Alkyl(hetero)cyclic compounds, process for their preparation and pharmaceutical compositions containing them

(30) Priorité: 11.01.1995 FR 9500238
(43) Date de publication de la demande: 17.07.1996
(73) Titulaire: ADIR ET COMPAGNIE, 92415 Courbevoie Cédex (FR)
(72) Inventeur: Lesieur, Daniel, F-59147 Gondecourt (FR); Fourmaintraux, Eric, F-62200 St. Martin/Boulonge /Mer (FR); Depreux, Patrick, F-59280 Armentieres (FR); Delagrange, Philippe, F-92130 Issy-les-Moulineaux (FR); Renard, Pierre, F-78000 Versailles (FR); Guardiola-Lemaître, Béatrice, F-92210 Saint-Cloud (FR)

(56) Documents cités:
- EP-A- 0 447 285
- EP-A- 0 527 687
- EP-A- 0 530 087
- EP-A- 0 562 956

## Description

L'invention concerne de nouveaux composés (hétéro)cycles alkylés, leur procédé de préparation et les compositions pharmaceutiques qui les contiennent.

L'invention décrit de nouveaux composés (hétéro)cycliques alkylés qui s'avèrent être de puissants ligands des récepteurs mélatoninergiques. On connaît dans l'art antérieur des composés décrits pour leurs propriétés mélatoninergiques comme par exemple dans les demandes de brevets EP 0 527 687, EP 0 562 956, EP 0 530 087 ou EP 0 447 285.

De nombreuses études ont mis en évidence ces dix dernières années, le rôle capital de la mélatonine (5-méthoxy N-acétyl tryptamine) dans le contrôle du rythme circadien et des fonctions endocrines, et les récepteurs de la mélatonine ont été caractérisés et localisés.

Outre leur action bénéfique sur les troubles du rythme circadien (J. Neurosurg 1985, 63, pp 321-341) et du sommeil (Psychopharmacology, 1990, 100, pp 222-226), les ligands du système mélatoninergique possèdent d'intéressantes propriétés pharmacologiques sur le système nerveux central, notamment anxiolytiques et antipsychotiques (Neuropharmacology of Pineal Secretions, 1990, 8 (3-4), pp 264-272) et analgésiques (Pharmacopsychiat., 1987, 20, pp 222-223) ainsi que pour le traitement de la maladie de Parkinson (J. Neurosurg 1985, 63, pp 321-341) et d'Alzheimer (Brain Research, 1990, 528, pp 170-174). De même, ces composés ont montré une activité sur certains cancers (Melatonin - clinical Perspectives, Oxford University Press, 1988, page 164-165), sur l'ovulation (Science 1987, 227, pp 714-720), et sur le diabète (Clinical endocrinolgy, 1986, 24, pp 359-364).

Des composés permettant d'agir sur le système mélatoninergique sont donc pour le clinicien d'excellents médicaments pour le traitement des pathologies mentionnées précédemment.

L'invention concerne les composés de formule (I): 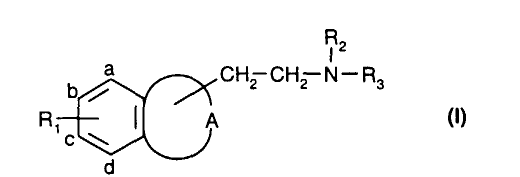 dans laquelle :
- R₁ représente un radical choisi parmi alkyle, alkyle substitué, cycloalkyle, cycloalkyle-substitué, cycloalkylalkyle, et cycloalkylalkyle substitué,
- A forme avec le noyau benzènique auquel il est lié un groupement cyclique choisi parmi benzofurane et 2,3-dihydrobenzofurane,
- R₂ représente un hydrogène ou un alkyle,
- R₃ représente :
   - un groupement R₃₁ : 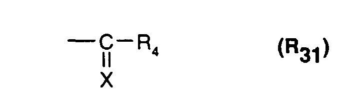 avec X représentant un soufre ou un oxygène et R₄ représentant un hydrogène ou un radical R₄₁ choisi parmi alkyle, alkyle substitué, alcényle, alcynyle, cycloalkyle, cycloalkyle substitué, cycloalkylalkyle et cycloalkylalkyle substitué,
   - ou un groupement de formule (R₃₂) : 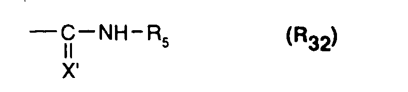 avec X' représentant un soufre ou un oxygène et R₅ représentant un hydrogène ou un radical choisi parmi alkyle, alkyle substitué, cycloalkyle, cycloalkyle substitué, cycloalkylalkyle et cycloalkylalkyle substitué,
   étant entendu que lors de la description de la formule (I), et sauf mention contraire :
- les termes "alkyle" et "alkoxy" désignent des groupements linéaires ou ramifiés contenant de 1 à 6 atomes de carbone,
- les termes "alcényle" et "alcynyle" désignent des groupements linéaires ou ramifiés contenant de 2 à 6 atomes,
- le terme "cycloalkyle" désigne un groupement de 3 à 8 atomes de carbone,
- le terme "substitué" associé au radical alkyle signifie que ce radical est substitué par un ou plusieurs substituants choisis parmi halogène, alkyle, hydroxy et alkoxy,
- le terme "substitué" associé au radical "cycloalkyle" et "cycloalkylalkyle" signifie que ce radical est substitué par un ou plusieurs radicaux ou groupements choisis parmi halogène, alkyle et oxo,
et leurs énantiomères et diastéréoisomères.

L'invention concerne particulièrement les composés de formule (I) dans laquelle, pris séparément ou ensemble,
- R₁ représente un alkyle,
- R₁ représente un (C₂-C₆) alkyle,
- R₁ représente un éthyle,
- R₁ représente un propyle,
- R₁ représente un butyle,
- A forme avec le noyau benzènique auquel il est lié un benzofurane,
- A forme avec le noyau benzènique auquel il est lié un 2,3-dihydrobenzofurane,
- R₂ représente un hydrogène,
- R₂ représente un alkyle,
- R₃ représente un groupement R₃₁ tel que défini dans la formule (I),
- R₃ représente un groupement R₃₂ tel que défini dans la formule (I),
- R₄ représente un atome d'hydrogène,
- R₄ représente un alkyle,
- R₄ représente un cycloalkyle,
- R₄ représente un alcényle,
- R₅ représente un hydrogène,
- R₅ représente un alkyle,
- R₅ représente un cycloalkyle,
- X représente un oxygène,
- X représente un soufre,
- X' représente un oxygène,
- ou X' représente un soufre.

Par exemple, l'invention concerne les composés particuliers de formule (I) répondant aux formules respectives (1) et (2) :

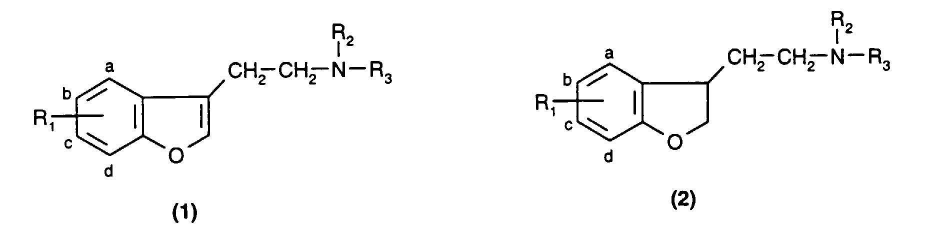

L'invention concerne particulièrement les composés de formule (I) par exemple les composés particuliers de formule (1) et (2) tels que définis ci-dessus dans laquelle R₁ est:
- en position a du noyau benzènique,
- en position b du noyau benzènique,
- en position c du noyau benzènique,
- ou en position d du noyau benzènique.

Par exemple, l'invention concerne les composés de formule (I) dans laquelle R₁ est en position b du noyau benzo.

De façon particulière, l'invention concerne les composés suivants :
- N-[2-(5-éthyl benzofuran-3-yl)éthyl]acétamide,
- N-[2-(5-éthyl benzofuran-3-yl)éthyl]cyclobutanecarboxamide,

De façon particulière, les radicaux alkyles présents dans la formule (I) peuvent être choisis parmi méthyle, éthyle, n-propyle, isopropyle, n-butyle, isobutyle, sec-butyle, tert-butyle, pentyle, ou hexyle.

Les radicaux alkoxy présents dans la formule (I) peuvent être choisis parmi méthoxy, éthoxy, n-propoxy, isopropoxy, n-butoxy, iso-butoxy, sec-butoxy, tert-butoxy, pentyloxy, et hexyloxy.

Les halogènes présents dans la formule (I) peuvent être choisis parmi le brome, le chlore, le fluor, et l'iode.

Les cycloalkyles présents dans la formule (I) peuvent être choisis parmi cyclopropyle, cyclobutyle, cyclopentyle, cyclohexyle, cycloheptyle et cyclooctyle.

L'invention concerne également le procédé de préparation des composés de formule (I) caractérisé en ce que : on fait réagir un composé de formule (II) : 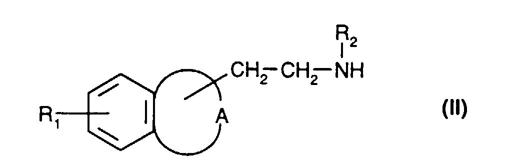 dans laquelle R_{1,} R₂ et A sont tels que définis dans la formule (I),
- soit avec de l'acide formique ou avec un composé de formule (IIIa) ou (IIIb) : 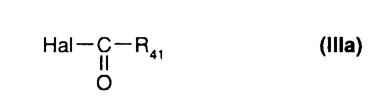 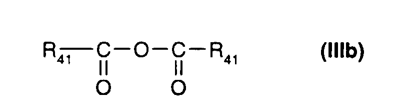 dans laquelle R₄₁ est tel que défini dans la formule (I) et Hal représente un halogène, afin d'obtenir les composés de formule (I/a): 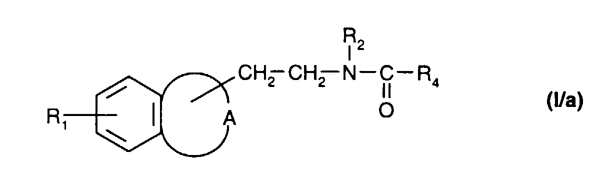 dans laquelle R₁, R₂, R₄ et A sont tels que définis précédemment,
   composés de formule (I/a) qui sont soumis au réactif de Lawesson pour obtenir les composés de formule (I/a'): 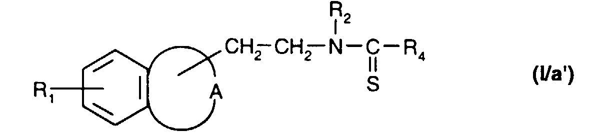 dans laquelle R₁, R₂, R₄ et A sont tels que définis précédemment,
- soit un composé de formule (IV) :

   X'=C=N―R₅ (IV)

   dans laquelle X' et R₅ sont tels que définis dans la formule (I)
   afin d'obtenir les composés de formule (I/b): 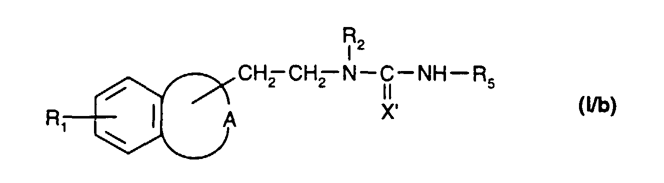 dans laquelle R₁, R₂, R₅, A et X' sont tels que définis précédemment,
les composés de formule (I/a), (I/a') et (I/b) formant l'ensemble des composés de formule (I), composés de formule (I) qui sont, le cas échéant, séparés en leurs différents énantiomères ou diastéréoisomères.

Par exemple, l'invention s'étend au procédé de préparation des composés de formule (I/c): 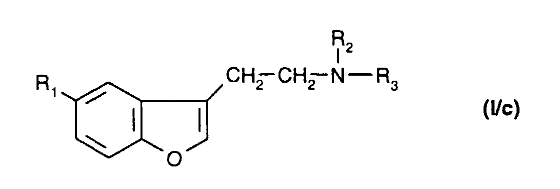 dans laquelle R₁, R₂, et R₃ sont tels que définis dans la formule (I),
caractérisé en ce que :
on fait réagir un composé de formule (II/b) : 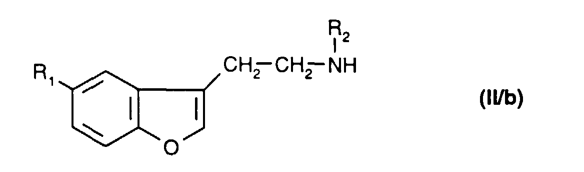 dans laquelle R₁, R₂ sont tels que définis précédemment,
- soit avec de l'acide formique soit avec un composé de formule (IIIa) ou (IIIb) tels que définis précédemment,
   afin d'obtenir les composés de formule (I/d) : 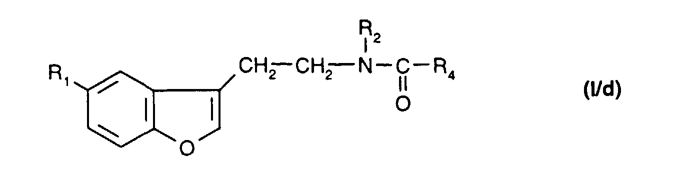 dans laquelle R₁, R₂ et R₄ sont tels que définis précédemment, qui sont ensuite soumis au réactif de Lawesson pour obtenir les composés de formule (I/d') : 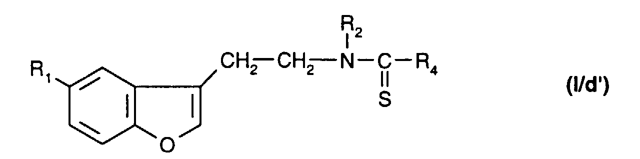 dans laquelle R₁, R₂ et R₄ sont tels que définis précédemment,
- soit avec un composé de formule (IV) tel que défini précédemment,
   afin d'obtenir les composés de formule (I/e) : 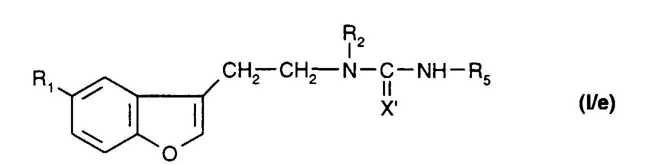 dans laquelle R₁, R₂, R₅ et X' sont tels que définis précédemment,
les composés de formule (I/d), (I/d') et (I/e) formant l'ensemble des composés de formule (I/c),
les composés de formule (I/c) pouvant être séparés en leurs différents énantiomères ou diastéréoisomères.

Les matières premières utilisées dans les procédés précédemment décrits sont soit commerciales, soit aisément accessibles à l'homme du métier d'après la littérature et les exemples de préparations données ci-après.

Par exemple, il est possible de préparer les composés de formule (Il/a) : 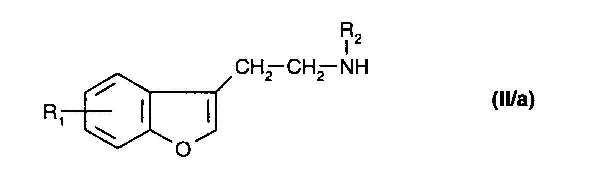 dans laquelle R₁ et R₂ sont tels que définis dans la formule (I),
par réaction d'un composé de formule (V) : 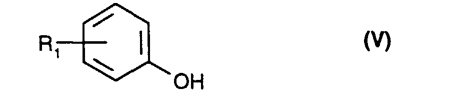 dans laquelle R₁ est tel que défini précédemment avec de l'anhydride acétique afin d'obtenir un composé de formule (VI) : 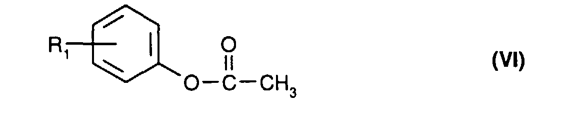 dans laquelle R₁ est tel que défini précédemment, composé de formule (VI) qui est mis en réaction avec un acide de Lewis pour obtenir un composé de formule (VII) : 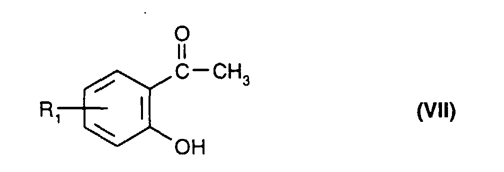 dans laquelle R₁ est tel que défini précédemment composé de formule (VII) qui est mis en réaction avec du bromure cuivrique pour obtenir le composé de formule (VIII) : 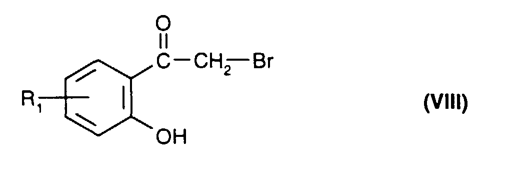 dans laquelle R₁ est tel que défini précédemment,
composé de formule (VIII) qui est cyclisé pour obtenir un composé de formule (IX) : 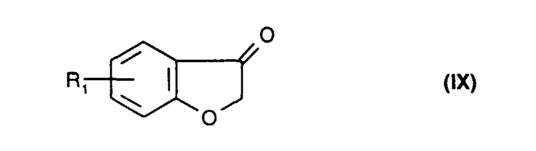 dans laquelle R₁ est tel que défini dans la formule (I), composé de formule (IX) qui est ensuite mis en réaction avec le cyanométhyl phosphonate de diéthyle en présence d'hydrure de sodium pour obtenir un composé de formule (X) : 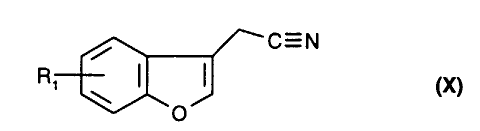 qui est ensuite hydrogéné et éventuellement alkylé sur l'azote pour obtenir un composé de formule (II/a) : 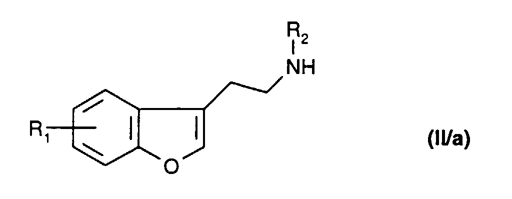 dans laquelle R₁ est tel que défini précédemment et les composés de formule (Il/a) pouvant être salifiés par un acide pharmaceutiquement acceptable.

Par exemple, il est possible de préparer les composés de formule (Il/b) : 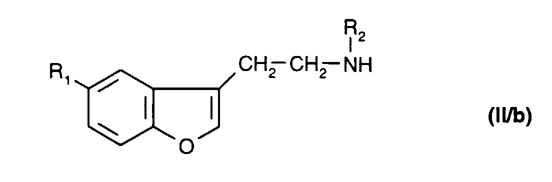 dans laquelle R₁ et R₂ sont tels que définirs dans la formule (I),
par réaction d'un composé de formule (V/b) : 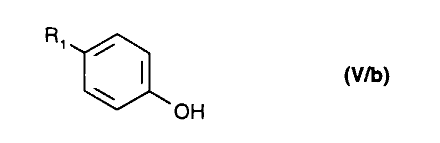 dans laquelle R₁ est tel que défini précédemment avec de l'anhydride acétique afin d'obtenir un composé de formule (VI/b) : 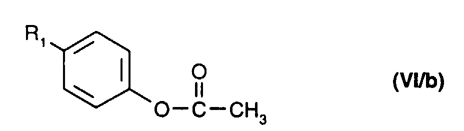 dans laquelle R₁ est tel que défini précédemment, composé de formule (VI/b) qui est mis en réaction avec un acide de Lewis pour obtenir un composé de formule (VII/b) : 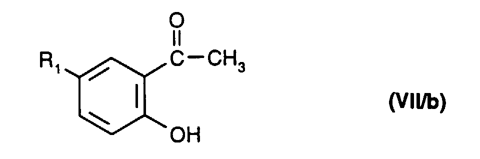 dans laquelle R₁ est tel que défini précédemment, composé de formule (Vll/b) qui est mis en réaction avec du bromure cuivrique pour obtenir le composé de formule (VIII/b) : 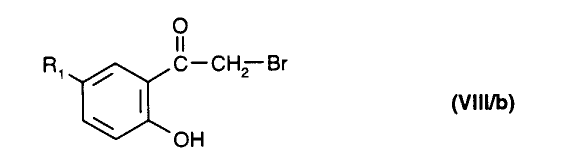 dans laquelle R₁ est tel que défini précédemment,
composé de formule (VIII/b) qui est cyclisé pour obtenir un composé de formule (IX/b) : 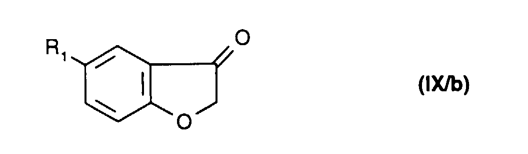 dans laquelle R₁ est tel que défini dans la formule (I), composé de formule (IX/b) qui est ensuite mis en réaction avec le cyanométhyl phosphonate de diéthyle en présence d'hydrure de sodium pour obtenir un composé de formule (X/b) : 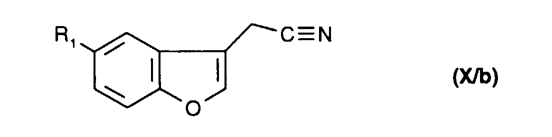 qui est ensuite hydrogéné et éventuellement alkylé sur l'azote pour obtenir un composé de formule (II/b) : 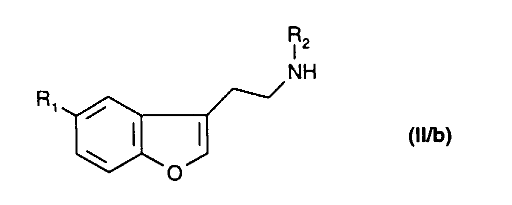 dans laquelle R₁ est tel que défini précédemment et les composés de formule (Il/b) pouvant être salifiés par un acide pharmaceutiquement acceptable.

Parmi les acides pharmaceutiquement acceptables que l'on peut utiliser pour former un sel d'addition avec les composés de formule (II), on peut citer à titre d'exemples et de façon non limitative, les acides chlorhydrique, sulfurique, phosphorique, tartrique, malique, maléique, fumarique, oxalique, méthanesulfonique, éthanesulfonique, camphorique, et citrique.

Les dérivés 2,3-dihydrobenzofurane nécessaires pour la préparation des composé de formule (I) dans laquelle A forme avec le cycle benzo auquel il est lié un 2,3-dihydrobenzofurane sont aisément accessibles à l'homme du métier par réduction ménagée du dérivé benzofurane correspondant.

L'invention s'étend également au procédé de préparation d'un composé de formule (I/f) : 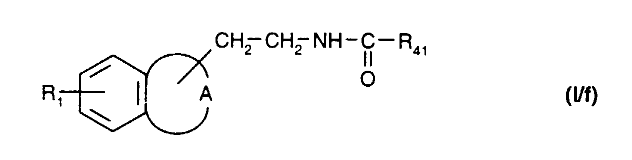 dans laquelle A, R₁ et R₄₁ sont tels que définis dans la formule (I),
par réaction, en présence de Nickel de Raney et d'hydrogène d'un dérivé de formule (XI) : 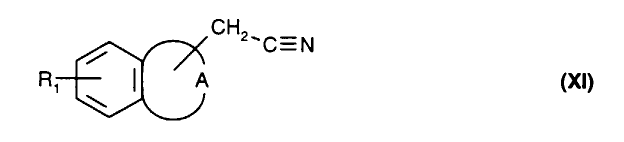 dans laquelle R₁ est tel que défini précédemment avec un composé de formule (III/a) ou (III/b) : 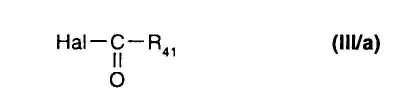 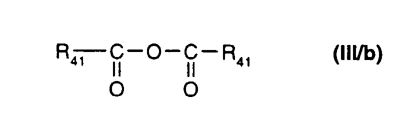 dans lesquelles R₄₁ est tel que défini précédemment et Hal représente un halogène.

Par exemple,
l'invention s'étend également au procédé de préparation d'un composé de formule (I/f') : 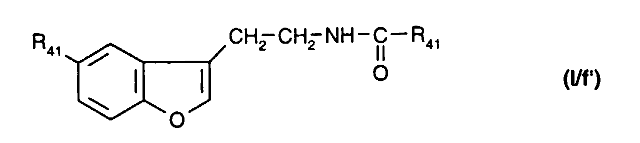 dans laquelle A, R₁ et R₄₁ sont tels que définis dans la formule (I),
par réaction, en présence de Nickel de Raney et d'hydrogène, d'un dérivé de formule (XI') : 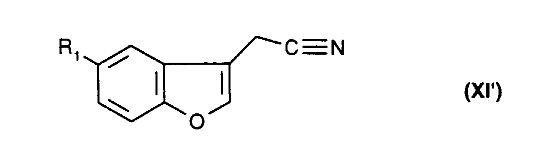 dans laquelle R₁ est tel que défini précédemment avec un composé de formule (III/a) ou (III/b) : 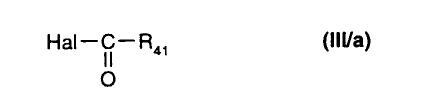 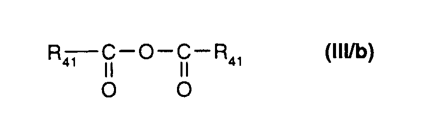 dans lesquelles R₄₁ est tel que défini précédemment et Hal représente un halogène.

Les composés de formule (I) possèdent des propriétés pharmacologiques très intéressantes pour le clinicien.

Les composés de l'invention et les compositions pharmaceutiques les contenant s'avèrent être utiles pour le traitement des troubles du système mélatoninergique.

L'étude pharmacologique des dérivés de l'invention a en effet montré qu'ils n'étaient pas toxiques, doués d'une très haute affinité sélective pour les récepteurs de la mélatonine et possédaient d'importantes activités sur le système nerveux central et en particulier, on a relevé des propriétés thérapeutiques sur les troubles du sommeil, des propriétés anxiolytiques, antipsychotiques, analgésiques ainsi que sur la microcirculation qui permettent d'établir que les produits de l'invention sont utiles dans le traitement du stress, des troubles du sommeil, de l'anxiété, des dépressions saisonnières, des pathologies cardiovasculaires, des insomnies et fatigues dues aux décalages horaires, de la schizophrénie, des attaques de panique, de la mélancolie, des troubles de l'appétit, de l'obésité, de l'insomnie, des troubles psychotiques, de l'épilepsie, de la maladie de Parkinson, de la démence sénile, des divers désordres liés au vieillissement normal ou pathologique, de la migraine, des pertes de mémoire, de la maladie d'Alzheimer, ainsi que les troubles de la circulation cérébrale. Dans un autre domaine d'activité, il apparaît que les produits de l'invention possèdent des propriétés d'inhibiteurs de l'ovulation, d'immunomodulateurs et qu'ils sont susceptibles d'être utilisés dans le traitement anticancéreux.

Les composés seront utilisés de préférence dans les traitements des dépressions saisonnières, des troubles du sommeil, des pathologies cardiovasculaires, des insomnies et fatigues dues aux décalages horaires, des troubles de l'appétit et de l'obésité.

Par exemple, les composés seront utilisés dans le traitement des dépressions saisonnières et des troubles du sommeil.

La présente invention a également pour objet les compositions pharmaceutiques contenant les produits de formule (I) en combinaison avec un ou plusieurs excipients pharmaceutiquement acceptables.

Parmi les compositions pharmaceutiques selon l'invention, on pourra citer, plus particulièrement celles qui conviennent pour l'administration orale, parentérale, nasale, per. ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques, et les ampoules buvables ou injectables.

La posologie varie selon le sexe, l'âge et le poids du patient, la voie d'administration, la nature de l'indication thérapeutique, ou des traitements éventuellement associés et s'échelonne entre 0,1 mg et 1 g par 24 heures en 1 ou 2 prises, plus particulièrement entre 1 à 100 mg, par exemple entre 1 à 10 mg.

Les exemples suivants illustrent l'invention, mais ne la limitent en aucune façon.

### PREPARATION 1: (5-ETHYL-BENZOFURAN-3-YL)ACETONITRILE

### STADE A: Acétate de 4-éthyl phényle

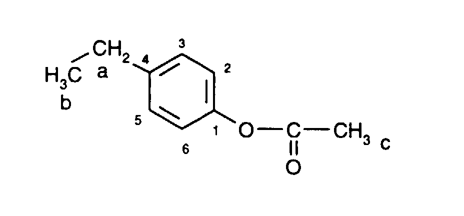

| **Réactifs :** | |
|---|---|
| 4-éthylphenol | 20 g |
| Anhydride acétique | : 100 ml |

### Mode opératoire :

Dans un erlen à col rodé de 250 ml, mélanger l'anhydride acétique avec le 4-éthylphénol. Laisser le mélange sous agitation pendant 5 heures.

Laisser le mélange revenir à température ambiante, puis le verser dans 1 litre d'eau et de glace pilée. Extraire trois fois à l'éther. Laver les phases éthérées plusieurs fois par une solution de K₂CO₃ 10% jusqu'à pH neutre. Sécher la phase éthérée sur CaCl₂ et porter à sec au rotavapor : on obtient une huile jaunâtre.

| **Caractéristiques :** | |
|---|---|
| PM | 164,20 g.Mol⁻¹ |
| Aspect | huile jaunâtre |
| Rendement | 84 % |

| **Analyse spectroscopique dans l'infrarouge :** | |
|---|---|
| 2840 à 3000 cm⁻¹ | υ CH |
| 1760 cm⁻¹ | υ C=O |

| **Analyse spectroscopique de RMN du proton (80 MHz, CDCl**_{**3**}**) :** | |
|---|---|
| δ = 1,15 ppm (triplet, 3H) | Hb |
| δ = 2,25 ppm (quadruplet, 2H) | Ha |
| δ = 2,65 ppm (singulet, 3H) | Hc |
| δ = 6,8 à 7,5 ppm (massif, 4H) | H aromatiques |

### STADE B : 5-éthyl-2-hydroxyacétophénone

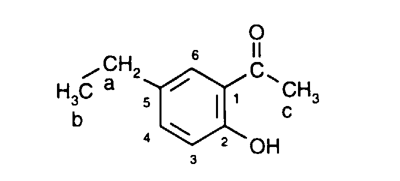

### Réactifs :

- acétate de 4-éthyl phényle : 19,36 g
- trichlorure d'aluminium : 38,8 g

### Mode opératoire :

Dans un ballon de 100 ml, mettre sous agitation l'ester et introduire par petites fractions AlCl₃ :
Placer le ballon dans un bain d'huile, préalablement chauffé à 100°C et laisser sous agitation pendant 1H30.

Verser le milieu réactionnel chaud sur 1 kg de glace pilée. Extraire trois fois à l'éther puis laver les phases organiques à l'eau jusqu'à pH neutre. Sécher la phase éthérée sur CaCl₂ et évaporer le solvant : on obtient une huile jaune.

| **Caractéristiques :** | |
|---|---|
| PM | 164,20 g.Mol⁻¹ |
| Aspect | huile jaune |
| Rendement | 89 % |

| **Analyse spectroscopique dans l'infrarouge :** | |
|---|---|
| 2840-3000 cm⁻¹ | υ CH |
| 1635 cm⁻¹ | υ C=O |

| **Analyse spectroscopique de RMN du proton (80 MHz, CDCl**_{**3**}**) :** | |
|---|---|
| δ = 1,20 ppm (triplet, 3H) | H_{b} |
| δ = 2,60 ppm (quadruplet, 2H) | Hₐ |
| δ = 2,60 ppm (singulet, 3H) | H_{c} |
| δ = 6,90 ppm (doublet, 1H) | H₃ Jo = 8,40 Hz |
| δ = 7,30 ppm (doublet, 1H) | H₆ Jm = 2,1 Hz |
| δ = 7,55 ppm (doublet dédoublet 1 H) | H₄ Jo = 8,40Hz ; Jm = 2,1 Hz |
| δ = 12,10 ppm (singulet, 1H) | OH |

### STADE C : 5-éthyl-2 hydroxy bromoacétophénone :

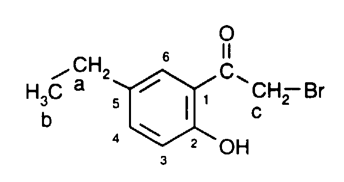

### Réactifs :

- 5-éthyl-2-hydroxy acétophénone : 18 g
- Bromure cuivrique (Cu Br₂) : 52,85 g
- Acétate d'éthyle - CHCl₃ (1-1)

### Mode opératoire :

Dans un erlen à col rodé de 250 ml, mélanger le bromure cuivrique et la 5-éthyl 2-hydroxyacétophénone dans le mélange acétate d'éthyle-CHCl₃ (90-90). Porter le milieu à reflux pendant 15 heures. Filtrer le précipité minéral, le rincer à l'acétate d'éthyle. Porter à sec le filtrat. Le résidu est repris dans l'acétate d'éthyle et rincé plusieurs fois à l'eau. Sécher la phase organique sur MgSO₄ et évaporer le solvant. Le résidu obtenu est alors purifié par chromatographie sur colonne de silice éluée par un mélange, CHCl₂-cyclohexane-toluène (4-4-2).

| **Caractéristiques :** | |
|---|---|
| PM | 243,11 g.Mol⁻¹ |
| Aspect | huile jaune |
| Rendement | 56 % |

| **Analyse spectroscopique dans l'infrarouge :** | |
|---|---|
| 2840-3000 cm⁻¹ | υ CH |
| 1630 cm⁻¹ | υ C=O |

| **Analyse spectroscopique de RMN du proton (80 MHz, CDCl**_{**3**}**) :** | |
|---|---|
| δ = 1,25 ppm (triplet, 3H) | H_{b} |
| δ = 2,60 ppm (quadruplet, 2H) | Hₐ |
| δ = 4,45 ppm (singulet, 2H) | H_{c} |
| δ = 6,9 ppm (doublet, 1H) | H₃ |
| δ = 7,4 ppm (doublet, 1 H) | H₆ |
| δ = 7,5 ppm (doublet, dédoublet 1H) | H₄ |
| δ = 11,6 ppm (singulet, 1H) | OH (échangeable dans D₂O) |

| **Microanalyse :** | | | |
|---|---|---|---|
| | % C | % H | %Br |
| % théorique | 49,41 | 4,56 | 32,87 |
| % trouvé | 49,08 | 4,34 | 33,19 |

### STADE D: 5-éthyl benzofuranone :

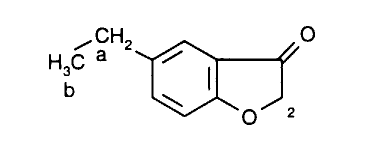

### Réactifs :

- 5-éthyl-2-hydroxy-bromoacétophénone : 5 g
- K₂CO₃ : 3,13 g
- Acétone : 50 ml

### Mode opératoire :

Dans un ballon de 100 ml, mélanger la 5-éthyl-2-hydroxy-bromoacétophénone dans l'acétone. Additionner le K₂CO₃ et laisser 4 heures sous agitation à température ambiante.

Essorer le minéral, le rincer à l'acétone et porter à sec le filtrat. Le résidu est alors purifié par chromatographie sur colonne de silice, élué par un mélange, cyclohexane-acétate d'éthyle. (9-1)

| **Caractéristiques :** | |
|---|---|
| PM | 162,19 g.Mol⁻¹ |
| Aspect | huile orangée |
| Rendement | 60 % |

| **Analyse spectroscopique dans l'infrarouge :** | |
|---|---|
| 2840-3000 cm⁻¹ | υ CH |
| 1700 cm⁻¹ | υ C=O |

| **Analyse spectroscopique de RMN du proton (300 MHz, CDCl**_{**3**}**) :** | |
|---|---|
| δ = 1,24 ppm (triplet, 3H) | H_{b} |
| δ = 2,66 ppm (quadruplet, 2H) | Hₐ |
| δ = 4,62 ppm (singulet, 2H) | H₂ |
| δ = 7,06 ppm (doublet, 1H) | H₇ |
| δ = 7,46 ppm (massif, 2H) | H₆ + H₄ |

### STADE E: (5-éthyl-benzofuran-3-yl)acétonitrile :

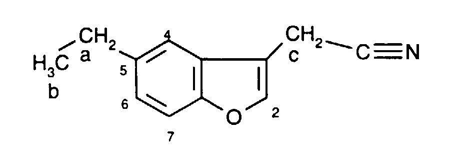

### Réactifs :

- 5-éthyl benzofuranone : 2 g
- Cyanométhyl phosphonate de diéthyle : 3,28 g
- Hydrure de sodium (60 %) (Na H) : 0,74 g
- Tétrahydrofurane (THF) : 25 ml

### Mode opératoire :

Dans un ballon bicol de 100 ml, mettre sous agitation et sous N₂, 15 ml de THF anhydre. Additionner par fraction NaH, puis par une ampoule à brome, additionner goutte à goutte le cyanométhyl-phosphonate de diéthyl. Laisser 1 heure sous agitation à température ambiante et sous azote. Additionner alors par l'ampoule la 5-éthylbenzofuranone, préalablement diluée dans 10 ml de THF. Laisser 1 heure sous agitation. Verser le milieu dans 250 ml d'eau et extraire trois fois à l'éther. Laver les phases éthérées à l'eau jusqu'à décoloration des eaux de lavage. Sécher la phase éthérée sur CaCl₂ et évaporer le solvant.

Le résidu huileux obtenu est alors purifié par chromatographie sur colonne de silice, élué par le mélange : cyclohexane-Acétate d'éthyle (9-1).

| **Caractéristiques :** | |
|---|---|
| PM | 185,23 g.Mol⁻¹ |
| Aspect | huile jaune |
| Rendement | 49 % |

| **Analyse spectroscopique dans l'infrarouge :** | |
|---|---|
| 2840-3000 cm⁻¹ | υ CH |
| 2240 cm⁻¹ | υ C ≡ N |

| **Analyse spectroscopique de RMN du proton(80 MHz, CDCl**_{**3**}**) :** | |
|---|---|
| δ = 1,26 ppm (triplet, 3H) | H_{b} |
| δ = 2,77 ppm (quadruplet, 2H) | Hₐ |
| δ = 3,72 ppm (singulet, 2H) | H₂ |
| δ = 7 à 7,75 ppm (massif, 3H) | H aromatiques |

| **Microanalyse :** | | | |
|---|---|---|---|
| | % C | % H | % N |
| théorique | 77,81 | 5,99 | 7,56 |
| trouvé | 77,53 | 6,18 | 7,15 |

### PREPARATIONS 2 A 6 :

En procédant comme dans la préparation 1 mais en utilisant au stade A le phénol convenablement substitué, on obtient les préparation suivantes :

### PREPARATION 2: (5-PROPYL-BENZOFURAN-3-YL)ACETONITRILE

### PREPARATION 3: (5-BUTYL-BENZOFURAN-3-YL)ACETONITRILE

### PREPARATION 4: (5-HEXYL-BENZOFURAN-3-YL)ACETONITRILE

### PREPARATION 5: (5-CYCLOPROPYL-BENZOFURAN-3-YL)ACETONITRILE

### PREPARATION 6: (5-CYCLOPROPYLMETHYL-BENZOFURAN-3-YL)ACETONITRILE

### PREPARATIONS 7 A 9 :

En soumettant les amines dérivées des préparations 1 à 3 à une réduction ménagée, on obtient les préparations suivantes.

### PREPARATION 7: N-[2-(5-ETHYL-2,3-DIHYDROBENZOFURAN-3-YL)ETHYL]AMINE

### PREPARATION 8: N-[2-(5-PROPYL-2,3-DIHYDRO-BENZOFURAN-3-YL)ETHYL]AMINE

### PREPARATION 9: N-[2-(5-BUTYL-2,3-DIHYDROBENZOFURAN-3-YL)ETHYL]AMINE

### PREPARATION 10: (6-ETHYL-BENZOFURAN-3-YL)ACETONITRILE

### EXEMPLE 1: N-[2-(5-ETHYLBENZOFURAN-3-YL)ETHYL]ACETAMIDE

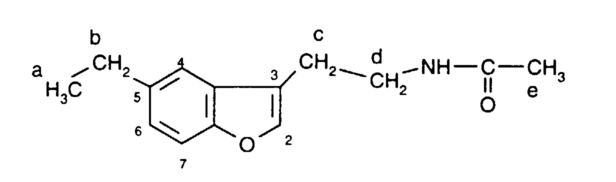

### Réactifs :

- (5-éthyl-benzofuran-3-yl)acétonitrile (préparation 1) : 4,45 g
- Anhydride acétique : 90 ml
- Nickel Raney (50 %) : 1,5 g
- H₂ : 60 bar

### Mode opératoire :

Dans une autoclave de 125 ml introduire le composé de la préparation 1 préalablement solubilisé dans l'anhydride acétique, le nickel de Raney. Mettre une pression d'hydrogène de 60 bar et laisser sous agitation à 50°C pendant 5 heures. Filtrer le nickel, le rincer à l'éthanol à 95° et porter à sec le filtrat. Reprendre le résidu dans 150 ml d'eau et alcaliniser par une solution NaOH 10 % jusqu'à pH 8. Laisser une heure sous agitation, puis extraire trois fois par l'acétate d'éthyle. Laver les phases organiques à l'eau jusqu'à pH neutre. Sécher la phase organique sur MgSO₄ et évaporer le solvant. Le résidu obtenu est alors purifié par chromatographie sur colonne de silice, élué par le mélange : acétone-toluène-cyclohexane (5-3-2). Le résidu obtenu est alors recristallisé dans le cyclohexane puis dans un mélange éther-éther de pétrol.

| **Caractéristiques :** | |
|---|---|
| PM | 231,28 g.Mol⁻¹ |
| Aspect | solide blanc |
| PF | 60-61 °C |
| Rendement | 50 % |

| **Analyse spectroscopique dans l'infrarouge :** | |
|---|---|
| 3290 cm⁻¹ | ν NH |
| 2840-3000 cm⁻¹ | ν CH |
| 1630 cm⁻¹ | ν C=O |

| **Analyse spectroscopique de RMN du proton (300 MHtz, CDCl**_{**3**}**) :** | |
|---|---|
| δ = 1,28 ppm (triplet, 3H) | Hₐ |
| δ = 1,96 ppm (singulet, 3H) | Hₑ |
| δ = 2,75 ppm (quadruplet, 2H) | H_{b} |
| δ = 2,90 ppm (triplet, 2H) | H_{c} |
| δ = 3,60 ppm (quadruplet, 2H) | H_{d} |
| δ = 5,55 ppm (signal, 1H) | NH |
| δ = 7,16 ppm (doublet dédoublet, 1H) | H₆ |
| δ = 7,44 ppm (massif, 2H) | H₇+H₄ |
| δ = 7,5 ppm (singulet, 2H) | H₂ |

| **Microanalyse :** | | | |
|---|---|---|---|
| | % C | % H | % N |
| théorique | 72,70 | 7,41 | 6,06 |
| trouvé | 72,45 | 7,50 | 6,13 |

### EXEMPLES 2 A 12 :

En procédant comme dans l'exemple 1, mais en utilisant le chlorure d'acyle ou l'anhydride d'acide approprié, on obtient le composé des exemples suivants :

### EXEMPLE 2: N-[2-(5-ETHYLBENZOFURAN-3-YL)ETHYL] 2-CHLOROACETAMIDE

### EXEMPLE 3: N-[2-(5-ETHYLBENZOFURAN-3-YL)ETHYL]PROPIONAMIDE

### EXEMPLE 4 : N-[2-(5-ETHYLBENZOFURAN-3-YL)ETHYL]BUTYRAMIDE

### EXEMPLE 5: N-[2-(5-ETHYLBENZOFURAN-3-YL)ETHYL]PENTANAMIDE

### EXEMPLE 6 : N-[2-(5-ETHYLBENZOFURAN-3-YL)ETHYL]HEXANAMIDE

### EXEMPLE 7: N-[2-(5-ETHYLBENZOFURAN-3-YL)ETHYL]HEPTANAMIDE

### EXEMPLE 8: N-[2-(5-ETHYLBENZOFURAN-3-YL)ETHYL]CYCLOPROPANE-CARBOXAMIDE

### EXEMPLE 9: N-[2-(5-ETHYLBENZOFURAN-3-YL)ETHYL]CYCLOBUTANE-CARBOXAMIDE

### EXEMPLE 10: N-[2-(5-ETHYLBENZOFURAN-3-YL)ETHYL]CYCLOPENTANE-CARBOXAMIDE

### EXEMPLE 11 : N-[2-(5-ETHYLBENZOFURAN-3-YL)ETHYL]CYCLOHEXANE-CARBOXAMIDE

### EXEMPLE 12: N-[2-(5-ETHYLBENZOFURAN-3-YL)ETHYL]TRIFLUORO-ACETAMIDE

### EXEMPLES 13 A 42:

En procédant comme dans l'exemple 1, mais en partant des préparations 2 à 6 et en utilisant les chlorures d'acyles et les anhydrides d'acides appropriés, on obtient les composés des exemples suivants .

### EXEMPLE 13: N-[2-(5-PROPYLBENZOFURAN-3-YL)ETHYL]ACETAMIDE

### EXEMPLE 14: N-[2-(5-PROPYLBENZOFURAN-3-YL)ETHYL]PROPIONAMIDE

### EXEMPLE 15: N-[2-(5-PROPYLBENZOFURAN-3-YL)ETHYL]BUTYRAMIDE

### EXEMPLE 16: N-[2-(5-PROPYLBENZOFURAN-3-YL)ETHYL]PENTANAMIDE

### EXEMPLE 17: N-[2-(5-PROPYLBENZOFURAN-3-YL)ETHYL]CYCLOPROPANECARBOXAMIDE

### EXEMPLE 18: N-[2-(5-PROPYLBENZOFURAN-3-YL)ETHYL]CYCLOBUTANECARBOXAMIDE

### EXEMPLE 19: N-[2-(5-BUTYLBENZOFURAN-3-YL)ETHYL]ACETAMIDE

### EXEMPLE 20: N-[2-(5-BUTYLBENZOFURAN-3-YL)ETHYL]PROPIONAMIDE

### EXEMPLE 21 : N-[2-(5-BUTYLBENZOFURAN-3-YL)ETHYL]BUTYRAMIDE

### EXEMPLE 22: N-[2-(5-BUTYLBENZOFURAN-3-YL)ETHYL]PENTANAMIDE

### EXEMPLE 23: N-[2-(5-BUTYLBENZOFURAN-3-YL)ETHYL]CYCLOPROPANECARBOXAMIDE

### EXEMPLE 24: N-[2-(5-BUTYLBENZOFURAN-3-YL)ETHYL]CYCLOBUTANECARBOXAMIDE

### EXEMPLE 25: N-[2-(5-HEXYLBENZOFURAN-3-YL)ETHYL]ACETAMIDE

### EXEMPLE 26: N-[2-(5-HEXYLBENZOFURAN-3-YL)ETHYL]PROPIONAMIDE

### EXEMPLE 27: N-[2-(5-HEXYLBENZOFURAN-3-YL)ETHYL]BUTYRAMIDE

### EXEMPLE 28: N-[2-(5-HEXYLBENZOFURAN-3-YL)ETHYL]PENTANAMIDE

### EXEMPLE 29: N-[2-(5-HEXYLBENZOFURAN-3-YL)ETHYL]CYCLOPROPANECARBOXAMIDE

### EXEMPLE 30: N-[2-(5-HEXYLBENZOFURAN-3-YL)ETHYL]CYCLOBUTANECARBOXAMIDE

### EXEMPLE 31 : N-[2-(5-CYCLOPROPYLBENZOFURAN-3-YL)ETHYL]ACETAMIDE

### EXEMPLE 32: N-[2-(5-CYCLOPROPYLBENZOFURAN-3-YL)ETHYL] PROPIONAMIDE

### EXEMPLE 33: N-[2-(5-CYCLOPROPYLBENZOFURAN-3-YL)ETHYL]BUTYRAMIDE

### EXEMPLE 34: N-[2-(5-CYCLOPROPYLBENZOFURAN-3-YL)ETHYL] PENTANAMIDE

### EXEMPLE 35: N-[2-(5-CYCLOPROPYLBENZOFURAN-3-YL)ETHYL]CYCLOPROPANECARBOXAMIDE

### EXEMPLE 36: N-[2-(5-CYCLOPROPYLBENZOFURAN-3-YL)ETHYL]CYCLOBUTANECARBOXAMIDE

### EXEMPLE 37: N-[2-(5-CYCLOPROPYLMETHYLBENZOFURAN-3-YL)ETHYL] ACETAMIDE

### EXEMPLE 38: N-[2-(5-CYCLOPROPYLMETHYLBENZOFURAN-3-YL)ETHYL] PROPIONAMIDE

### EXEMPLE 39: N-[2-(5-CYCLOPROPYLMETHYLBENZOFURAN-3-YL)ETHYL] BUTYRAMIDE

### EXEMPLE 40: N-[2-(5-CYCLOPROPYLMETHYLBENZOFURAN-3-YL)ETHYL] PENTANAMIDE

### EXEMPLE 41 : N-[2-(5-CYCLOPROPYLMETHYLBENZOFURAN-3-YL)ETHYL] CYCLOPROPANECARBOXAMIDE

### EXEMPLE 42: N-[2-(5-CYCLOPROPYLMETHYLBENZOFURAN-3-YL)ETHYL] CYCLOBUTANECARBOXAMIDE

### EXEMPLES 43 A 47 :

En utilisant l'amine obtenue par hydrogénation de la préparation 1 et le dérivé isocyanate ou isothiocyanate approprié, on obtient les composés des exemples suivants.

### EXEMPLE 43: N-[2-(5-ETHYLBENZOFURAN-3-YL)ETHYL] N'-METHYLUREE

### EXEMPLE 44: N-[2-(5-ETHYLBENZOFURAN-3-YL)ETHYL] N'-ETHYLUREE

### EXEMPLE 45: N-[2-(5-ETHYLBENZOFURAN-3-YL)ETHYL] N'-PROPYLUREE

### EXEMPLE 46: N-[2-(5-ETHYLBENZOFURAN-3-YL)ETHYL] N'-CYCLOPROPYLUREE

### EXEMPLE 47: N-[2-(5-ETHYLBENZOFURAN-3-YL)ETHYL] N'-CYCLOBUTYLUREE

### EXEMPLES 48 A 52 :

En utilisant l'amine obtenue par hydrogénation de la préparation 2 et le dérivé isocyanate ou isothiocyanate approprié, on obtient les composés des exemples suivants.

### EXEMPLE 48: N-[2-(5-PROPYLBENZOFURAN-3-YL)ETHYL] N'-METHYLUREE

### EXEMPLE 49: N-[2-(5-PROPYLBENZOFURAN-3-YL)ETHYL] N'-ETHYLUREE

### EXEMPLE 50: N-[2-(5-PROPYLBENZOFURAN-3-YL)ETHYL] N'-PROPYLUREE

### EXEMPLE 51: N-[2-(5-PROPYLBENZOFURAN-3-YL)ETHYL] N'-CYCLOPROPYLUREE

### EXEMPLE 52: N-[2-(5-PROPYLBENZOFURAN-3-YL)ETHYL] N'-CYCLOBUTYLUREE

### EXEMPLES 53 A 55 :

En procédant comme dans l'exemple 1, mais en partant des préparations 7 à 9, on obtient les composés des exemples suivants.

### EXEMPLE 53: N-[2-(5-ETHYL-2,3-DIHYDROBENZOFURAN-3-YL)ETHYL] ACETAMIDE

### EXEMPLE 54: N-[2-(5-PROPYL-2,3-DIHYDROBENZOFURAN-3-YL)ETHYL] ACETAMIDE

### EXEMPLE 55: N-[2-(5-BUTYL-2,3-DIHYDROBENZOFURAN-3-YL)ETHYL] ACETAMIDE

### EXEMPLE 56: N-[2-(6-ETHYL-BENZOFURAN-3-YL)ETHYL]ACETAMIDE

### ETUDE PHARMACOLOGIQUE

### EXEMPLE A: ETUDE DE LA TOXICITE AIGUE

La toxicité aiguë a été appréciée après administration orale à des lots de 8 souris (26 ± 2 grammes). Les animaux ont été observés à intervalles réguliers au cours de la première journée et quotidiennement pendant les deux semaines suivant le traitement. La DL 50, entraînant la mort de 50 % des animaux, a été évaluée.

La DL 50 des produits testés est supérieure à 1000 mg.kg⁻¹ pour les composés étudiés ce qui indique la faible toxicité des composés de l'invention.

### EXEMPLE B: ETUDE DE LIAISON AUX RECEPTEURS DE LA MELATONINE

### B1) ETUDE SUR DES CELLULES DE LA PARS TUBERALIS DE MOUTON

Les études de liaison aux récepteurs de la mélatonine des composés de l'invention ont été réalisées selon les techniques classiques sur les cellules de la pars tuberalis de mouton. La pars tuberalis de l'adénohypophyse est en effet caractérisée, chez les mammifères, par une haute densité en récepteurs de la mélatonine (Journal of Neuroendocrinology vol. (1), pp 1-4 (1989)).

### PROTOCOLE

1) Les membranes de pars Tuberalis de mouton sont préparées et utilisées comme tissu cible dans des expériences de saturation pour déterminer les capacités et affinités de liaison pour la 2-[¹²⁵I]-iodomélatonine.
2) Les membranes de Pars tuberalis de mouton sont utilisées comme tissu cible, avec les différents composés à tester, dans des expériences de liaison compétitive par rapport à la 2-[¹²⁵I]-mélatonine.

Chaque expérience est réalisée en triple et une gamme de concentrations différentes est testée pour chaque composé.

Les résultats permettent de déterminer, après traitement statistique, les affinités de liaison du composé testé.

### RESULTATS

Il apparaît que les composés de l'invention possèdent une puissante affinité pour les récepteurs de la mélatonine supérieure à la mélatonine elle-même.

### B2) ETUDE SUR DES MEMBRANES DE CELLULES DU CERVEAU DE POULET (GALLUS DOMESTICUS)

Les animaux utilisés sont des poulets (Gallus domesticus) agés de 12 jours. Ils sont sacrifiés entre 13 et 17 heures le jour de leur arrivée. Les cerveaux sont rapidement prélevés et congelés à - 200°C puis conservés à - 80°C. Les membranes sont préparées selon la méthode décrite par Yuan et Pang (Journal of Endocrinology 128, pages 475-482, 1991). La 2-[¹²⁵I] mélatonine est incubée en présence des membranes dans une solution tamponnée à pH 7.4 pendant 60 min à 25°C. A l'issue de cette période, la suspension membranaire est filtrée (Whatman GF/C). La radioactivité retenue sur le filtre est déterminée à l'aide d'un compteur à scintillation liquide Beckman® LS 6000.

Les produits utilisés sont :
- 2-[¹²⁵I] mélatonine
- mélatonine
- produits courants
- molécules originales

En screening primaire, les molécules sont testées à 2 concentrations (10⁻⁷ et 10⁻⁵ M). Chaque résultat est la moyenne de n=3 mesures indépendantes. Les molécules actives retenues d'après les résultats du screening primaire ont fait l'objet d'une détermination quantitative de leur efficacité (IC₅₀). Elles sont utilisées à 10 concentrations différentes.

Ainsi les valeurs d'IC₅₀ trouvées pour les composés préférés de l'invention, qui correspondent aux valeurs de l'affinité montrent que la liaison des composés testés est très puissante.

### EXEMPLE C : TEST DES QUATRE PLAQUES

Les produits de l'invention sont administrés par voie oesophagienne à des lots de dix souris. Un lot reçoit du sirop de gomme. 30 minutes après l'administration des produits à étudier, les animaux sont placés dans des habitacles dont le plancher comprend quatre plaques métalliques. Chaque fois que l'animal passe d'une plaque à l'autre, il reçoit une légère décharge électrique (0,35 mA). Le nombre de passages est enregistré pendant une minute. Après administration, les composés de l'invention augmentent de façon significative le nombre de passages ce qui montre l'activité anxiolytique des dérivés de l'invention.

### EXEMPLE D: COMPOSES DE L'INVENTION SUR LES RYTHMES CIRCADIENS D'ACTIVITE LOCOMOTRICE DU RAT

L'implication de la mélatonine dans l'entraînement, par l'alternance jour/nuit, de la plupart des rythmes circadiens physiologiques, biochimiques et comportementaux a permis d'établir un modèle pharmacologique pour la recherche de ligands mélatoninergiques.

Les effets des molécules sont testés sur de nombreux paramètres et en particulier sur les rythmes circadiens d'activité locomotrice qui représentent un marqueur fiable de l'activité de l'horloge circadienne endogène.

Dans cette étude, on évalue les effets de telles molécules sur un modèle expérimental particulier, à savoir le rat placé en isolement temporel (obscurité permanente).

### PROTOCOLE EXPERIMENTAL

Des rats mâles Long Evans âgés de un mois sont soumis dès leur arrivée au laboratoire à un cycle lumineux de 12h de lumière par 24h (LD 12 : 12).

Après 2 à 3 semaines d'adaptation, ils sont placés dans des cages équipées d'une roue reliée à un système d'enregistrement afin de détecter les phases d'activité locomotrice et de suivre ainsi les rythmes nycthéméraux (LD) ou circadiens (DD).

Dès que les rythmes enregistrés témoignent d'un entraînement stable par le cycle lumineux LD 12 : 12, les rats sont mis en obscurité permanente (DD).

Deux à trois semaines plus tard, lorsque le libre-cours (rythme reflétant celui de l'horloge endogène) est clairement établi, les rats reçoivent une administration quotidienne de la molécule à tester.
Les observations sont réalisées grâce à la visualisation des rythmes d'activité :
- entraînement des rythmes d'activité par le rythme lumineux,
- disparition de l'entraînement des rythmes en obscurité permanente,
- entraînement par l'administration quotidienne de la molécule ; effet transitoire ou durable.

Un logiciel permet :
- de mesurer la durée et l'intensité de l'activité, la période du rythme chez les animaux en libre cours et pendant le traitement,
- de mettre éventuellement en évidence par analyse spectrale l'existence de composants circadiens et non circadiens (ultradiens par exemple).

### RESULTATS :

Il apparaît clairement que les composés de l'invention permettent d'agir de façon puissante sur le rythme circadien via le système mélatoninergique.

### EXEMPLE E: ACTIVITE ANTIARYTHMIQUE

### PROTOCOLE

### (Ref : LAWSON J.W. et al. J. Pharmacol. Expert. Therap. 160 : 22-31, 1968)

La substance testée est administrée en intrapéritonéal à un groupe de 3 souris 30 min avant l'exposition à une anesthésie par le chloroforme. Les animaux sont ensuite observés pendant 15 min. L'absence d'enregistrement d'arythmies et de fréquences cardiaques supérieures à 200 battements / min (témoin : 400-480 battements / min) chez deux animaux au moins indique une protection significative.

### EXEMPLE F: ACTIVITE ANTI-AGREGANTE PLAQUETTAIRE

### PROTOCOLE

(Ref.: Bertele V. et al. Science. 220 : 517-519, 1983
Ibid, Eur. J. Pharmacol, 85 : 331-333, 1982)

Les composés de l'invention (100 µg/ml) sont testés pour leur capacité d'inhiber l'agrégation plaquettaire irréversible induite par l'arachidonate de sodium (50 µg/ml) dans du plasma de lapin enrichi en plaquettes.

Une inhibition de plus de 50 % de l'agrégation maximum indique une activité significative pour les composés de l'invention.

Ce test *in vitro* montre que les composés de l'invention sont de bons candidats pour le traitement des maladies cardiovasculaires, notamment les thromboses

### EXEMPLE G : PROLONGATION DU TEMPS DE SAIGNEMENT

### PROTOCOLE

(Ref.: Djana E. et al. Thrombosis Research. 15 : 191-197, 1979)
Butler K.D. et al. Thromb. Haemostasis. 47 : 46-49, 1982)

Les composés à tester sont administrés par voie orale (100 mg/kg) à un groupe de 5 souris 1h avant le sectionnement standardisé du bout de chaque queue (0,5 mm).

Les souris sont immédiatement suspendues verticalement, les queues étant immergées de 2 cm dans un tube à essai contenant une solution saline isotonique à 37°C.

Le temps requis pour que le saignement cesse pendant une période de 15 secondes est alors déterminé.

Une prolongation de plus de 50 % du temps de saignement relative à un groupe d'animaux contrôle est considérée comme significative pour les composés de l'invention.

Ce test *in vivo* confirme l'intérêt des composés de l'invention pour le traitement des pathologies cardiovasculaires puisque les composés de l'invention prolongent le temps de saignement.

### EXEMPLE H: TEST D'HYPOXIE HYPOBARE

### PROTOCOLE

(Ref.: Gotti B., et Depoortere H., Circ. Cerebrale, Congrès de Circulation Cérébrale,
Toulouse, 105-107, 1979)

Les composés à tester sont administrés par voie intrapéritonéale (100 mg/kg) à un groupe de 3 souris 30 minutes avant d'être placés dans une chambre à la pression hypobare de 20 cm Hg.

La prolongation du temps de survie par rapport à un groupe d'animaux traités avec le véhicule de plus de 100 % en absence d'effet dépresseur du système nerveux central indique une activité cérébroprotective des composés de l'invention.

### EXEMPLE I : COMPOSITION PHARMACEUTIQUE : COMPRIMES

1000 comprimés dosés à 5 mg de N-[2-(5-éthyl-benzofuran-3-yl)éthyl]acétamide

| | |
|---|---|
| N-[2-(5-éthyl-benzofuran-3-yl)éthyl]acétamide | 5 g |
| Amidon de blé | 20 g |
| Amidon de maïs | 20 g |
| Lactose | 30 g |
| Stéarate de magnésium | 2 g |
| Silice | 1 g |
| Hydroxypropylcellulose | 2 g |

## Revendications

1. Composés de formule (I) : 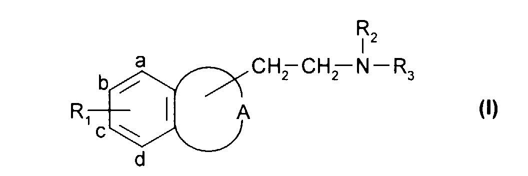 dans laquelle :
- R₁ représente un radical choisi parmi alkyle, alkyle substitué, cycloalkyle, cycloalkyle substitué, cycloalkylalkyle, et cycloalkylalkyle substitué,
- A forme avec le noyau benzènique auquel il est lié un groupement cyclique choisi parmi benzofurane et 2,3-dihydrobenzofurane,
- R₂ représente un hydrogène ou un alkyle,
- R₃ représente :
• un groupement R₃₁ : 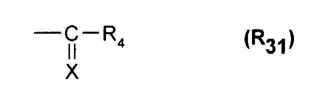 avec X représentant un soufre ou un oxygène et R₄ représentant un hydrogène ou un radical R₄₁ choisi parmi alkyle, alkyle substitué, alcényle, alcynyle, cycloalkyle, cycloalkyle substitué, cycloalkylalkyle et cycloalkylalkyle substitué,
• ou un groupement de formule (R₃₂) : 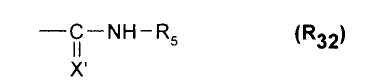 avec X' représentant un soufre ou un oxygène et R₅ représentant un hydrogène ou un radical choisi parmi alkyle, alkyle substitué, cycloalkyle, cycloalkyle substitué, cycloalkylalkyle et cycloalkylalkyle substitué,
étant entendu que lors de la description de la formule (I), et sauf mention contraire :
- les termes "alkyle" et "alkoxy" désignent des groupements linéaires ou ramifiés contenant de 1 à 6 atomes de carbone,
- les termes "alcényle" et "alcynyle" désignent des groupements linéaires ou ramifiés contenant de 2 à 6 atomes de carbone,
- le terme "cycloalkyle" désigne un groupement de 3 à 8 atomes de carbone,
- le terme "substitué" associé au radical alkyle signifie que ce radical est substitué par un ou plusieurs substituants choisis parmi halogène, alkyle, hydroxy et alkoxy,
- le terme "substitué" associé au radical "cycloalkyle" et "cycloalkylalkyle" signifie que ce radical est substitué par un ou plusieurs radicaux ou groupements choisis parmi halogène, alkyle et oxo,
et leurs énantiomères et diastéréoisomères.

2. Composés de formule (I) selon la revendication 1 dans laquelle, pris séparément ou ensemble,
- R₁ représente un alkyle,
- R₁ représente un (C₂-C₆)alkyle,
- R₁ représente un éthyle,
- R₁ représente un propyle,
- R₁ représente un butyle,
- A forme avec le noyau benzènique auquel il est lié un benzofurane,
- A forme avec le noyau benzènique auquel il est lié un 2,3-dihydrobenzofurane,
- R₂ représente un hydrogène,
- R₂ représente un alkyle,
- R₃ représente un groupement R₃₁ tel que défini dans la formule (I),
- R₃ représente un groupement R₃₂ tel que défini dans la formule (I),
- R₄ représente un atome d'hydrogène,
- R₄ représente un alkyle,
- R₄ représente un cycloalkyle,
- R₄ représente un alcényle,
- R₅ représente un hydrogène,
- R₅ représente un alkyle,
- R₅ représente un cycloalkyle,
- X représente un oxygène,
- X représente un soufre,
- X' représente un oxygène,
- ou X' représente un soufre.

3. Composés de formule (I) selon la revendication 1 répondant aux formules respectives (1) et (2) :

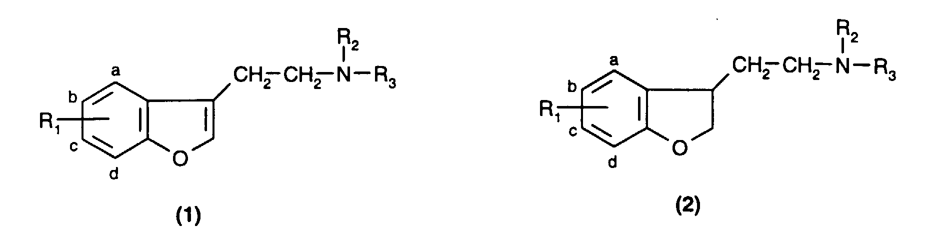

4. Composés de formule (I) selon la revendication 1 dans laquelle R₁ est en position b du noyau benzo.

5. Composé de formule (I) selon la revendication 1 qui est le N-[2-(5-éthyl benzofuran-3-yl)éthyl]acétamide.

6. Procédé de préparation des composés de formule (I) selon la revendication 1 caractérisé en ce que :
on fait réagir un composé de formule (II) : 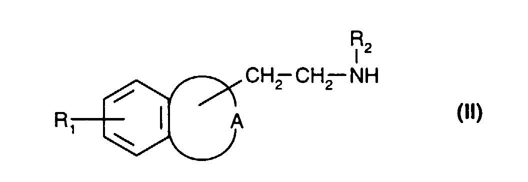 dans laquelle R_{1,} R₂ et A sont tels que définis dans la revendication 1,
- soit avec de l'acide formique ou avec un composé de formule (IIIa) ou (IIIb): 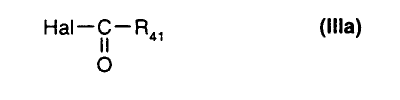 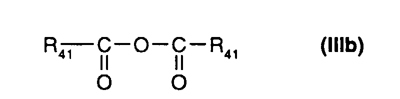 dans laquelle R₄₁ est tel que défini dans la revendication 1 et Hal représente un halogène, afin d'obtenir les composés de formule (I/a): 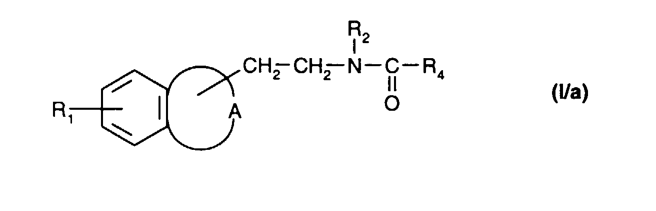 dans laquelle R₁, R₂, R₄ et A sont tels que définis précédemment,
composés de formule (I/a) qui sont soumis au réactif de Lawesson pour obtenir les composés de formule (I/a'): 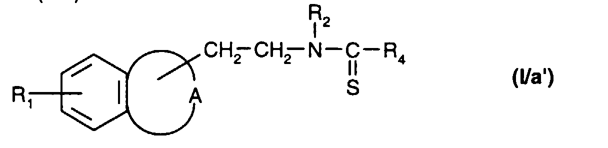 dans laquelle R₁, R₂, R₄ et A sont tels que définis précédemment,
- soit un composé de formule (IV):
X'=C=N―R₅ (IV)
dans laquelle X' et R₅ sont tels que définis dans la revendication 1 afin d'obtenir les composés de formule (I/b): 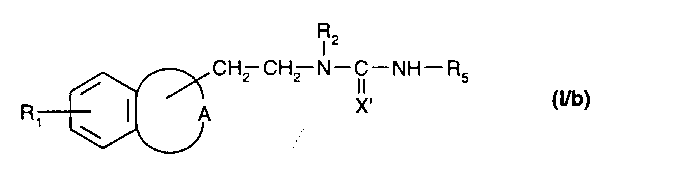 dans laquelle R₁, R₂, R₅, A et X' sont tels que définis précédemment,
les composés de formule (I/a), (I/a') et (I/b) formant l'ensemble des composés de formule (I), composés de formule (I) qui sont, le cas échéant, séparés en leurs différents énantiomères ou diastéréoisomères.

7. Procédé de préparation d'un composé selon la revendication 1 de formule (I/f) : 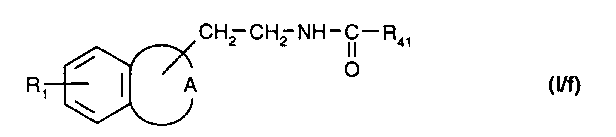 dans laquelle A, R₁ et R₄₁ sont tels que définis dans la revendication 1,
par réaction, en présence de Nickel de Raney et d'hydrogène d'un dérivé de formule (XI) : 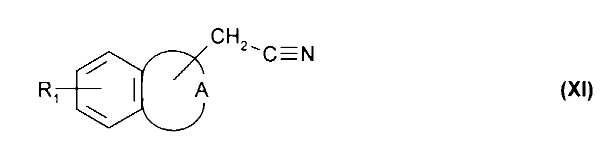 dans laquelle R₁ est tel que défini précédemment avec un composé de formule (III/a) ou (III/b) : 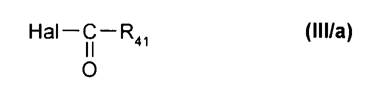 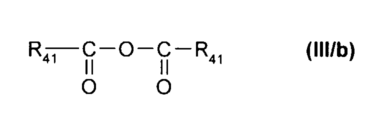 dans lesquelles R₄₁ est tel que défini précédemment.

8. Compositions pharmaceutiques contenant les produits de formule (I) selon la revendication 1 en combinaison avec un ou plusieurs excipients pharmaceutiquement acceptables.

9. Compositions pharmaceutiques selon la revendication 8 utiles pour le traitement des troubles du système mélatoninergique.

10. Compositions pharmaceutiques selon la revendication 8 dans les traitements des dépressions saisonnières, des troubles du sommeil, des pathologies cardiovasculaires, des insomnies et fatigues dues aux décalages horaires, des troubles de l'appétit et de l'obésité.

## Claims

1. Compounds of formula (I) : 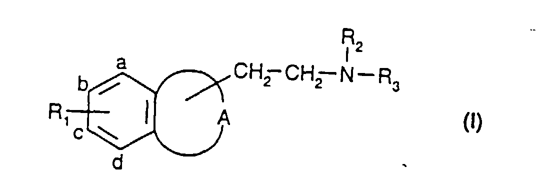 wherein
- R₁ represents a radical selected from alkyl, substituted alkyl, cycloalkyl, substituted cycloalkyl, cycloalkylalkyl and substituted cycloalkylalkyl,
- A, together with the benzene nucleus to which it is attached, forms a cyclic group selected from benzofuran and 2,3-dihydrobenzofuran,
- R₂ represents hydrogen or alkyl,
- R₃ represents :
• a group R₃₁ : 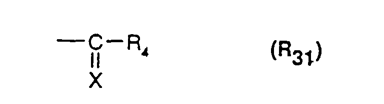 wherein X represents sulphur or oxygen and R₄ represents hydrogen or a radical R₄₁ selected from alkyl, substituted alkyl, alkenyl, alkynyl, cycloalkyl, substituted cycloalkyl, cycloalkylalkyl and substituted cycloalkylalkyl,
• or a group of formula (R₃₂) 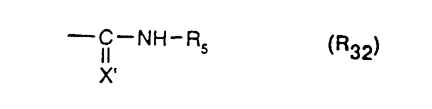 wherein X' represents sulphur or oxygen and R₅ represents hydrogen or a radical selected from alkyl, substituted alkyl, cycloalkyl, substituted cycloalkyl, cycloalkylalkyl and substituted cycloalkylalkyl,
wherein in the description of formula (I), unless specified otherwise :
- the terms "alkyl" and "alkoxy" denote linear or branched groups containing from 1 to 6 carbon atoms,
- the terms "alkenyl" and "alkynyl" denote linear or branched groups containing from 2 to 6 carbon atoms,
- the term "cycloalkyl" denotes a group containing from 3 to 8 carbon atoms,
- the term "substituted" associated with an alkyl radical denotes that that radical is substituted by one or more substituents selected from halogen, alkyl, hydroxy and alkoxy,
- the term "substituted" associated with the radicals "cycloalkyl" and "cycloalkylalkyl" denotes that such radicals are substituted by one or more radicals or groups selected from halogen, alkyl and oxo,
and their enantiomers and diastereoisomers.

2. Compounds of formula (I) according to claim 1 wherein, taken separately or together,
- R₁ represents alkyl,
- R₁ represents (C₂-C₆)alkyl,
- R₁ represents ethyl,
- R₁ represents propyl,
- R₁ represents butyl,
- A, together with the benzene nucleus to which it is attached, forms benzofuran,
- A, together with the benzene nucleus to which it is attached, forms 2,3-dihydrobenzofuran,
- R₂ represents hydrogen,
- R₂ represents alkyl,
- R₃ represents a group R₃₁ as defined for formula (I),
- R₃ represents a group R₃₂ as defined for formula (I),
- R₄ represents a hydrogen atom,
- R₄ represents alkyl,
- R₄ represents cycloalkyl,
- R₄ represents alkenyl,
- R₅ represents hydrogen,
- R₅ represents alkyl,
- R₅ represents cycloalkyl,
- X represents oxygen,
- X represents sulphur,
- X' represents oxygen,
- or X' represents sulphur.

3. Compounds of formula (I) according to claim 1 corresponding to the respective formulae (1) and (2):

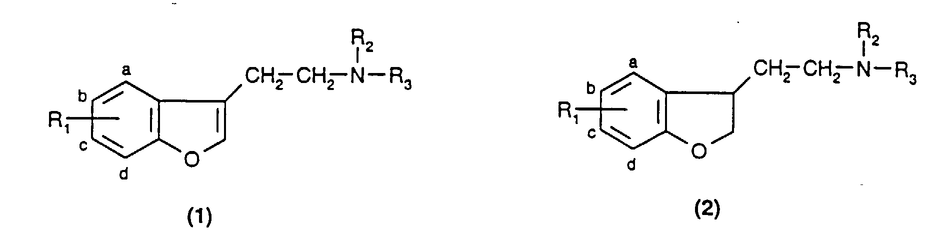

4. Compounds of formula (I) according to claim 1, wherein R₁ is in the b position of the benzo nucleus.

5. Compound of formula (I) according to claim 1 that is N-[2-(5-ethylbenzofuran-3-yl)ethyl]acetamide.

6. Process for the preparation of the compounds of formula (I) according to claim 1, characterised in that:
a compound of formula (II) : 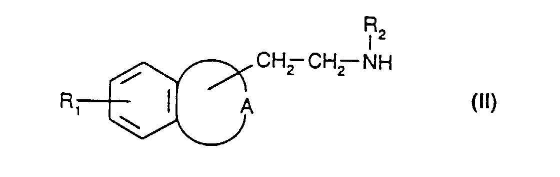 wherein R₁, R₂ and A are as defined in claim 1 is reacted
- either with formic acid or with a compound of formula (IIIa) or (IIIb) : 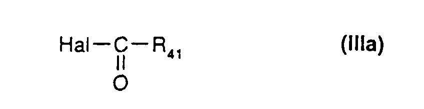 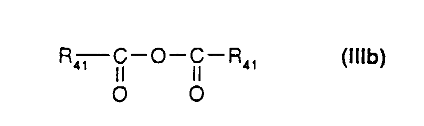 wherein R₄₁ is as defined in claim 1 and Hal represents halogen, to obtain compounds of formula (I/a): 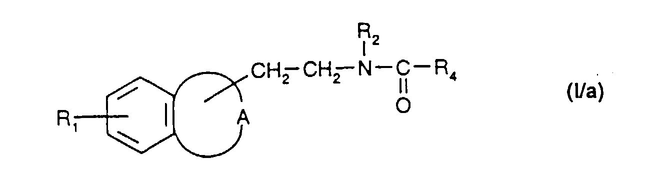 wherein R₁, R₂, R₄ and A are as defined above,
which compounds of formula (I/a) are subjected to Lawesson's reagent to obtain compounds of formula (I/a'): 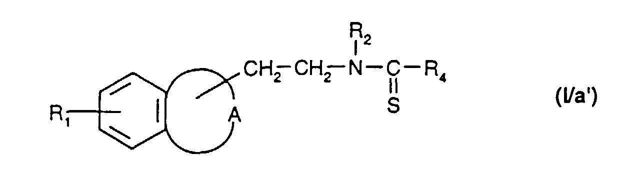 wherein R₁, R₂, R₄ and A are as defined above,
- or with a compound of formula (IV):
X'=C=N―R₅ (IV)
wherein X' and R₅ are as defined in claim 1,
to obtain compounds of formula (I/b): 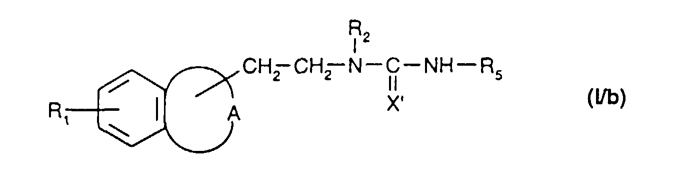 wherein R₁, R₂, R₅, A and X' are as defined above,
the compounds of formulae (I/a), (I/a') and (I/b) constituting the totality of the compounds of formula (I), which compounds of formula (I) are, if necessary, separated into their different enantiomers or diastereoisomers.

7. Process for the preparation of a compound according to claim 1 of formula (I/f): 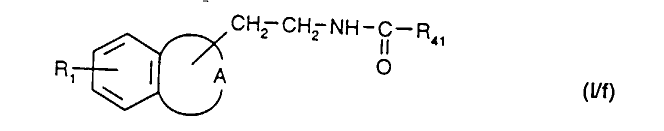 wherein A, R₁ and R₄₁ are as defined in claim 1,
by reaction, in the presence of Raney nickel and hydrogen, of a compound of formula (XI): 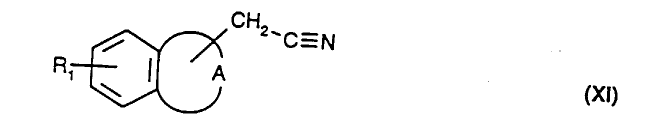 wherein R₁ is as defined above, with a compound of formula (III/a) or (III/b) : 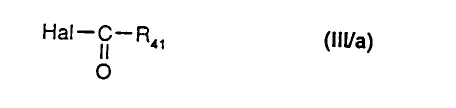 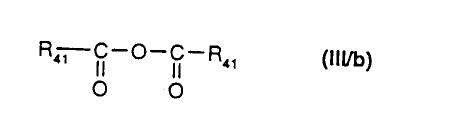 wherein R₄₁ is as defined above.

8. Pharmaceutical compositions comprising the products of formula (I) according to claim 1 in combination with one or more pharmaceutically acceptable excipients.

9. Pharmaceutical compositions according to claim 8 for use in the treatment of disorders of the melatoninergic system.

10. Pharmaceutical compositions according to claim 8 in the treatment of seasonal affective disorder, sleep disorders, cardiovascular pathologies, insomnia and fatigue caused by jet-lag, appetite disorders and obesity.

## Patentansprüche

1. Verbindungen der Formel (I): 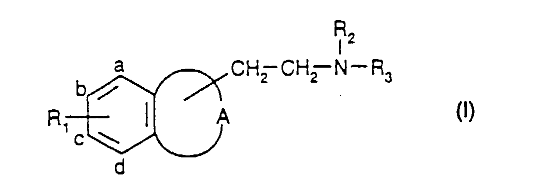 in der:
- R₁ eine Gruppe ausgewählt aus Alkyl, substituiertem Alkyl, Cycloalkyl, substituiertem Cycloalkyl, Cycloalkylalkyl und substituiertem Cycloalkylalkyl bedeutet,
- A zusammen mit dem Benzolkern, an den es gebunden ist, eine cyclische Gruppe bildet, ausgewählt aus Benzofuran und 2,3-Dihydrobenzofuran,
- R₂ Wasserstoff oder Alkyl bedeutet,
- R₃:
• eine Gruppe R₃₁: 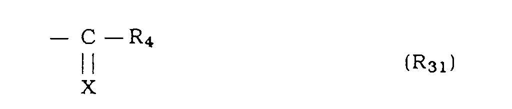 in der X Schwefel oder Sauerstoff und R₄ Wasserstoff oder eine Gruppe R₄₁ ausgewählt aus Alkyl, substituiertem Alkyl, Alkenyl, Alkinyl, Cycloalkyl, substituiertem Cycloalkyl, Cycloalkylalkyl und substituiertem Cycloalkylalkyl bedeuten,
• oder eine Gruppe der Formel (R₃₂) darstellt: 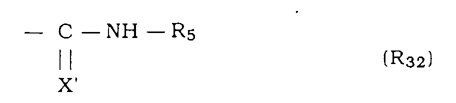 worin X' Schwefel oder Sauerstoff und R₅ Wasserstoff oder eine Gruppe ausgewählt aus Alkyl, substituiertem Alkyl, Cycloalkyl, substituiertem Cycloalkyl, Cycloalkylalkyl und substituiertem Cycloalkylalkyl bedeuten,
mit der Maßgabe, daß, wenn nichts anderes erwähnt ist, in der Beschreibung der Formel (I):
- die Begriffe "Alkyl" und "Alkoxy" für geradkettige oder verzweigte Gruppen, die 1 bis 6 Kohlenstoffatome enthalten, stehen,
- die Begriffe "Alkenyl" und "Alkinyl" für geradkettige oder verzweigte Gruppen, die 2 bis 6 Kohlenstoffatome enthalten, stehen,
- der Begriff "Cycloalkyl" für eine Gruppe mit 3 bis 8 Kohlenstoffatomen steht,
- der Begriff "substituiert" in Kombination mit dem Alkylrest bedeutet, daß dieser Rest durch einen oder mehrere Substituenten ausgewählt aus Halogen, Alkyl, Hydroxy und Alkoxy substituiert ist,
- der Begriff "substituiert" in Kombination mit einem "Cycloalkyl"- und "Cycloalkylalkyl"-Rest bedeutet, daß dieser Rest durch einen oder mehrere Reste oder Gruppen ausgewählt aus Halogen, Alkyl und Oxo substituiert ist,
und deren Enantiomere und Diastereoisomere.

2. Verbindungen der Formel (I) nach Anspruch 1, worin getrennt oder gemeinsam:
- R₁ Alkyl bedeutet,
- R₁ (C₂-C₆)-Alkyl bedeutet,
- R₁ Ethyl bedeutet,
- R₁ Propyl bedeutet,
- R₁ Butyl bedeutet,
- A mit dem Benzolkern, an den es gebunden ist, Benzofuran bildet,
- A mit dem Benzolkern, an den es gebunden ist, 2,3-Dihydrobenzofuran bildet,
- R₂ Wasserstoff bedeutet,
- R₂ Alkyl bedeutet,
- R₃ eine Gruppe R₃₁ bedeutet, wie sie bezüglich der Formel (I) definiert worden ist,
- R₃ eine Gruppe R₃₂ bedeutet, wie sie bezüglich der Formel (I) definiert worden ist,
- R₄ Wasserstoff bedeutet,
- R₄ Alkyl bedeutet,
- R₄ Cycloalkyl bedeutet,
- R₄ Alkenyl bedeutet,
- R₅ Wasserstoff bedeutet,
- R₅ Alkyl bedeutet,
- R₅ Cycloalkyl bedeutet,
- X Sauerstoff bedeutet,
- X Schwefel bedeutet,
- X' Sauerstoff bedeutet,
- oder X' Schwefel bedeutet.

3. Verbindungen der Formel (I) nach Anspruch 1 entsprechend den Formeln (1) und (2):

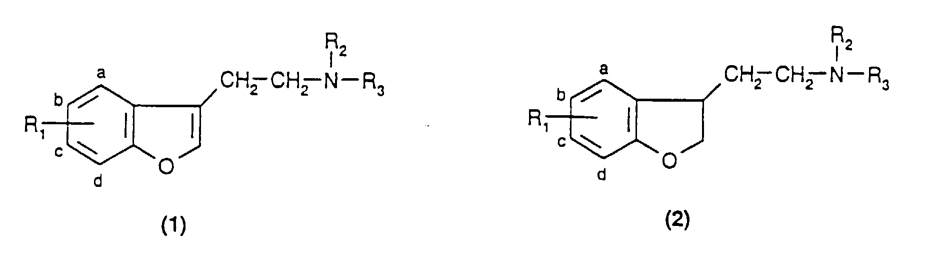

4. Verbindungen der Formel (I) nach Anspruch 1, worin R₁ in der Stellung b des Benzolkerns steht.

5. Verbindung der Formel (I) nach Anspruch 1, nämlich N-[2-(5-Ethyl-benzofuran-3-yl)-ethyl]-acetamid.

6. Verfahren zur Herstellung der Verbindungen der Formel (I) nach Anspruch 1, dadurch gekennzeichnet, daß man:
eine Verbindung der Formel (II): 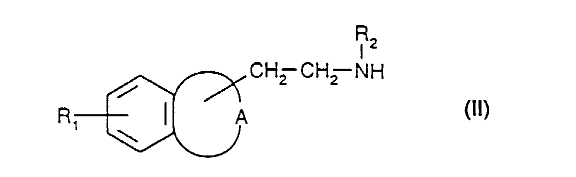 in der R₁, R₂ und A die in Anspruch 1 angegebenen Bedeutungen besitzen,
- entweder mit Ameisensäure oder mit einer Verbindung der Formel (IIIa) oder (IIIb): 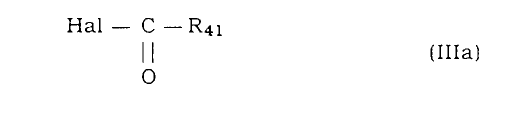 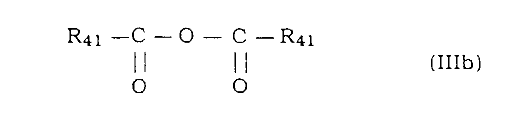 in der R₄₁ die in Anspruch 1 angegebenen Bedeutungen besitzen und Hal Halogen bedeutet, umsetzt zur Bildung der Verbindungen der Formel (I/a): 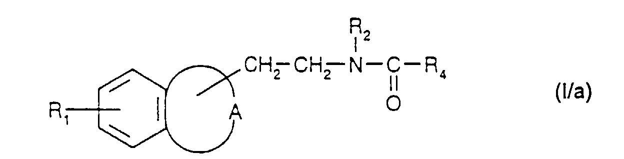 in der R₁, R₂, R₄ und A die oben angegebenen Bedeutungen besitzen,
welche Verbindungen der Formel (I/a) der Einwirkung des Lawesson-Reagens unterworfen werden zur Bildung der Verbindungen der Formel (I/a'): 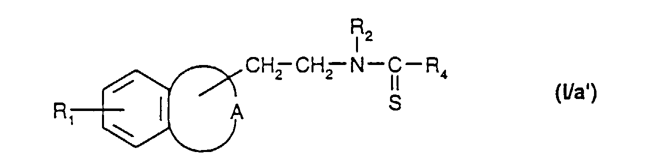 in der R₁, R₂, R₄ und A die oben angegebenen Bedeutungen besitzen,
- oder mit einer Verbindung der Formel (IV):
X' = C = N ― R₅ (IV)
in der X' und R₅ die in Anspruch 1 angegebenen Bedeutungen besitzen, umsetzt zur Bildung der Verbindungen der Formel (I/b): 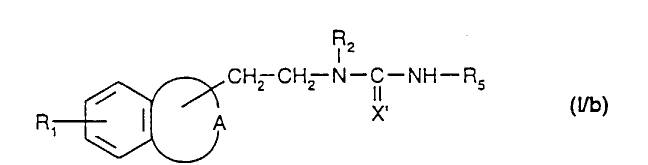 in der R₁, R₂, R₅, A und X' die oben angegebenen Bedeutungen besitzen, welche Verbindungen der Formeln (I/a), (I/a') und (I/b) die Gesamtheit der Verbindungen der Formel (I) bilden, welche Verbindungen der Formel (I) gegebenenfalls in ihre verschiedenen Enantiomeren oder Diastereoisomeren aufgetrennt werden.

7. Verfahren zur Herstellung einer Verbindung nach Anspruch 1 der Formel (I/f): 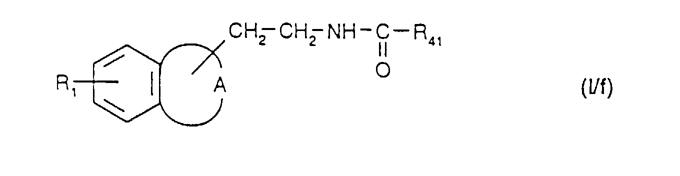 in der A, R₁ und R₄₁ die in Anspruch 1 angegebenen Bedeutungen besitzen, durch Umsetzen eines Derivats der Formel (XI): 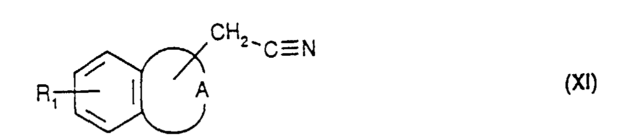 in der R₁ die oben angegebenen Bedeutungen besitzt, mit einer Verbindung der Formel (III/a) oder (III/b): 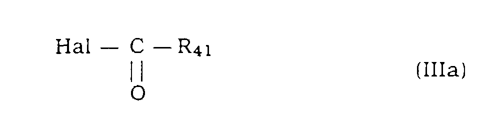 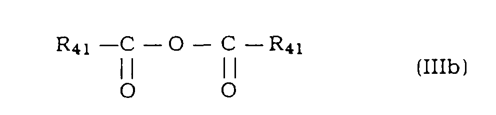 worin R₄₁ die oben angegebenen Bedeutungen besitzt, in Gegenwart von Raney-Nickel und Wasserstoff.

8. Pharmazeutische Zubereitungen enthaltend die Produkte der Formel (I) nach Anspruch 1 in Kombination mit einem oder mehreren pharmazeutisch annehmbaren Trägermaterialien.

9. Pharmazeutische Zubereitungen nach Anspruch 8 zur Behandlung von Störungen des melatoninergischen Systems.

10. Pharmazeutische Zubereitungen nach Anspruch 8 zur Behandlung von saisonal bedingten Depressionen, Schlafstörungen, kardiovaskulären pathologischen Zuständen, Schlaflosigkeit und Müdigkeit als Folge von Zeitverschiebungen, Appetitstörungen und Fettsucht.
